(19) **Europäisches Patentamt**
**European Patent Office**
**Office européen des brevets**

(11) **EP 1 867 302 B1**

(12) **FASCICULE DE BREVET EUROPEEN**

(45) Date de publication et mention
de la délivrance du brevet:
**09.09.2009 Bulletin 2009/37**

(51) Int Cl.:
***A61F 2/38*** (2006.01)

(21) Numéro de dépôt: **07290700.9**

(22) Date de dépôt: **06.06.2007**

(54) **Prothèse tricompartiment à renfort**

Dreiteilige Stützprothese.

Three-compartment prosthesis with reinforcement.

(84) Etats contractants désignés:
**DE FR GB IT**

(30) Priorité: **12.06.2006 FR 0605171**

(43) Date de publication de la demande:
**19.12.2007 Bulletin 2007/51**

(73) Titulaires:
• **Biegun, Jean-Francois**
 **90800 Bavilliers (FR)**
• **X.NOV**
 **90000 Belfort (FR)**

(72) Inventeurs:
• **Biegun, Jean-Francois**
 **90800 Bavilliers (FR)**
• **Marceaux, Pascal**
 **52000 Chaumont (FR)**

(74) Mandataire: **Eidelsberg, Olivier Nathan et al**
 **Cabinet Aymard & Coutel,**
 **22 Avenue de Friedland**
 **75008 Paris (FR)**

(56) Documents cités:
**EP-A1- 0 294 298     DE-A1- 4 041 920**
**US-A- 4 081 866      US-A- 4 353 135**

**Description**

La présente invention se rapporte à un composant fémoral d'une prothèse de genou, ainsi qu'à une prothèse de genou comportant un composant fémoral de ce genre.

Il est connu, dans le domaine des prothèses de genou, de nombreux types de composants fémoraux, par exemple US-4081866 A. Ces composants fémoraux actuels comportent généralement deux condyles du côté postérieur et une partie de trochlée du côté antérieur, à la surface extérieure de laquelle est défini un trajet pour la trochlée. L'articulation du genou est très complexe. Plusieurs mouvements cohabitent au niveau de cette articulation lors de la flexion du tibia, le fémur tournant et glissant par rapport à la partie tibiale, de même que la rotule qui glisse et a un mouvement de translation sur la trochlée. Il est donc nécessaire, pour assurer une cinématique parfaite de la rotule sur le fémur tout en évitant des phénomènes de luxation, que les trajets de trochlée puissent être bien définis dans la partie de trochlée, et notamment celle-ci être doit être très creuse à sa surface extérieure pour définir le trajet de la trochlée. Les prothèses doivent être très épaisses pour pouvoir accepter un tel creusement de la partie de trochlée. En outre, dans la partie intérieure du composant fémoral, un ensemble de faces planes définissent une cavité. Pour pouvoir réaliser une partie de trochlée dans laquelle un trajet de trochlée est défini suffisamment profondément, il est nécessaire aujourd'hui de faire en sorte que cette cavité comporte au moins cinq faces planes. La combinaison de la grande épaisseur nécessaire du composant fémoral (épaisseur entre sa surface extérieure et la surface intérieure définie par les faces planes intérieures de la cavité), et du fait d'avoir à réaliser cinq faces planes pour former la cavité intérieure, pour pouvoir accepter un chemin de trochlée très creusé, fait que, lors de la pose de la prothèse, le chirurgien doit réséquer beaucoup de l'os fémoral. Or, il est très néfaste de retirer beaucoup d'os. Le fémur s'en trouve affaibli et, lors d'une éventuelle reprise de la prothèse, le capital osseux n'est généralement plus suffisant et il est alors nécessaire d'utiliser un implant très invasif, ce qui nécessite une opération lourde et onéreuse.

La présente invention vise à surmonter les inconvénients des prothèses de l'art antérieur en proposant un composant fémoral qui, tout en étant moins épais, permet cependant d'avoir une partie de trochlée aussi creusée que dans le cas des prothèses de l'art antérieur pour avoir ainsi un trajet de trochlée qui s'étend aussi profondément dans la surface extérieure de la prothèse que dans le cas de prothèses de l'art antérieur, de sorte qu'on obtient une aussi bonne cinématique de la prothèse tout en ayant à effectuer moins de résection de l'os lors de la pose du composant fémoral.

Suivant l'invention, un composant fémoral d'une prothèse de genou, comportant au moins un, de préférence deux condyles, du côté postérieur ; une partie de trochlée du côté antérieur, dans la surface supérieure de laquelle est défini un trajet de trochlée ; et un ensemble de faces planes intérieures, destinées à venir en contact avec des faces réséquées correspondantes de l'extrémité d'un fémur, définissant une cage ouverte intérieure à la surface intérieure du composant, l'ensemble de faces planes comportant une première face plane d'extrémité antérieure, dite coupe antérieure, une deuxième face plane intermédiaire, dite coupe distale, et une troisième face plane d'extrémité postérieure, dite coupe postérieure, est caractérisé en ce qu'il est formé, entre la face plane d'extrémité antérieure et la face plane intermédiaire, une surface bombée ayant sa concavité tournée vers l'extérieur du composant.

En prévoyant ainsi cette partie bombée entre la partie d'extrémité antérieure, c'est-à-dire sensiblement la partie de trochlée, et la face intermédiaire, on peut ainsi augmenter le trajet de trochlée qui, après s'être étendu sur toute la partie d'extrémité antérieure, peut se poursuivre le long de la surface bombée. On obtient ainsi un trajet de trochlée aussi long que dans le cas des prothèses de l'art antérieur et, cependant, il n'est plus nécessaire de réaliser un composant fémoral aussi épais que dans l'art antérieur, dans lequel notamment il était nécessaire de prévoir une face supplémentaire entre la face d'extrémité antérieure et la face intermédiaire.

Suivant un perfectionnement de l'invention, la surface bombée est symétrique par rapport au plan antéro-postérieur et la courbe définissant la section transversale dans le plan antéro-postérieur de cette surface bombée, au niveau du trajet de la trochlée définie dans la partie de trochlée, est en continuité avec ce même trajet de trochlée, et notamment a le même rayon de courbure et le même centre de courbure.

Suivant un perfectionnement de l'invention, la deuxième face plane intermédiaire fait un angle par rapport à la perpendiculaire à la première face plane d'extrémité antérieure supérieur à 4°, de préférence compris entre 6° et 8°.

Suivant un perfectionnement de l'invention, la deuxième face plane intermédiaire fait un angle par rapport à la perpendiculaire à la troisième face plane d'extrémité postérieure supérieur à 4°, de préférence compris entre 6° et 8°.

Suivant un perfectionnement de l'invention, la deuxième face plane intermédiaire forme, avec chacune des première et troisième faces planes d'extrémités antérieure et postérieure, des coins, en étant contiguë avec celles-ci.

Suivant un perfectionnement de l'invention, la surface bombée qui fait saillie à l'intérieur de la cavité a, en coupe dans le plan médiolatéral passant par le plan de la première face plane d'extrémité antérieure, la forme d'une courbe qui est incluse strictement à l'intérieur de la cavité intérieure, de sorte que deux segments de droite de part et d'autre de cette surface bombée forment des arêtes de coin entre la face intermédiaire et la face d'extrémité antérieure.

Suivant un mode de réalisation préféré de l'invention, il est disposé dans les coins formés entre la ou les troisième face(s) d'extrémité postérieure(s) et la deuxième face intermédiaire des renforts.

La présente invention se rapporte également à une prothèse de genou comportant un composant fémoral suivant l'invention et une partie tibiale, un insert tibial étant disposé entre la partie tibiale et le composant fémoral.

On décrit maintenant, à titre d'exemple uniquement, un mode de réalisation de l'invention, en se reportant aux dessins dans lesquels :

la Figure 1 est une vue en perspective de dessus d'un composant fémoral suivant l'invention ;

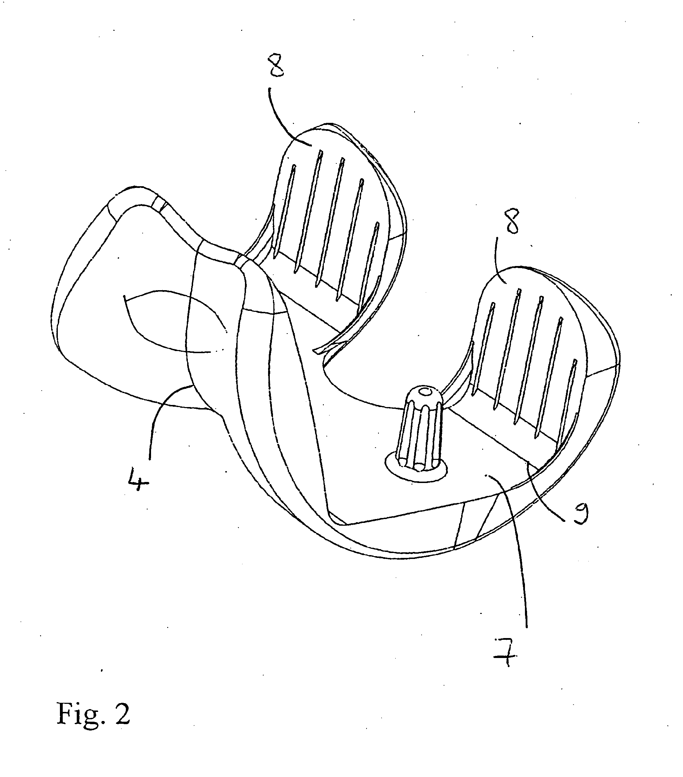

la Figure 2 est une vue en perspective suivant un autre angle du composant fémoral de la Figure 1 ;
la Figure 3 est une vue de côté du composant fémoral des Figures 1 et 2 ;

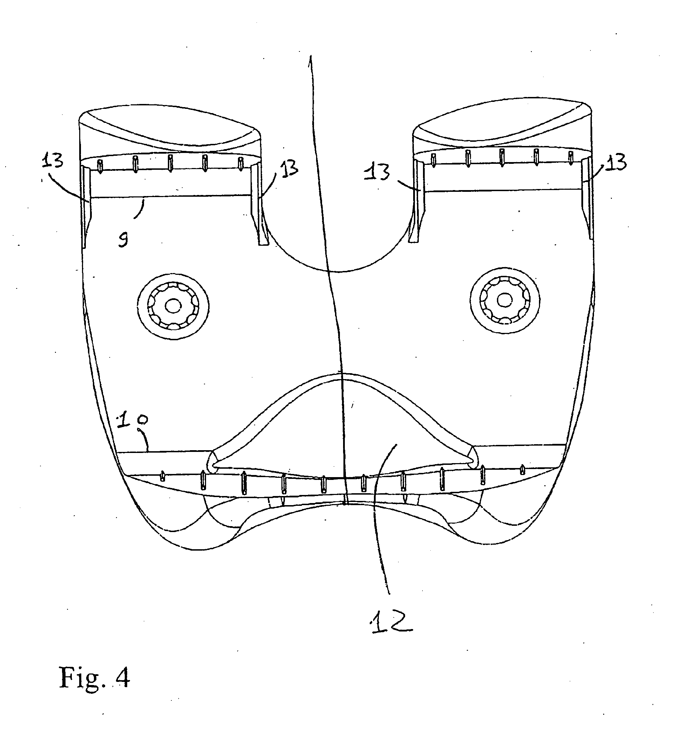

la Figure 4 est une vue de dessus du composant fémoral des Figures 1 à 3 ;

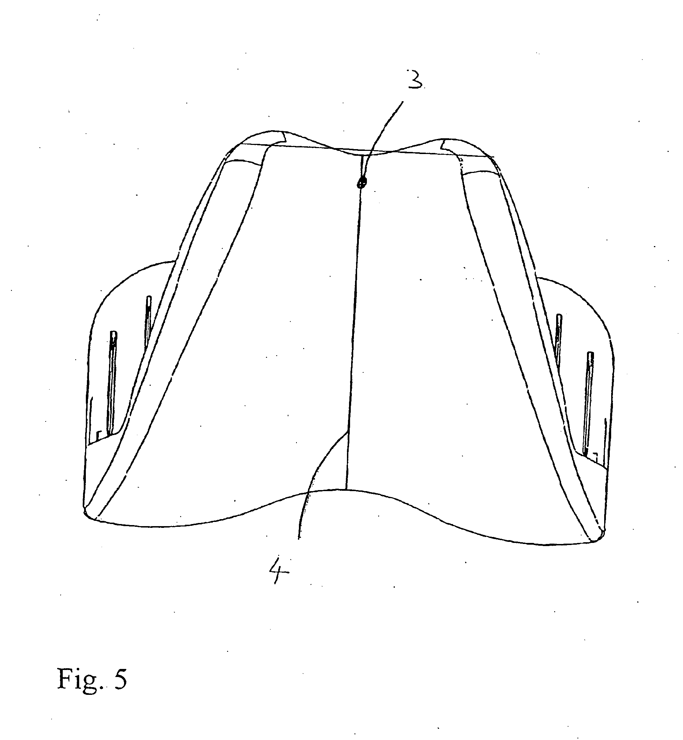

la Figure 5 est une vue de face du composant fémoral des Figures 1 à 4.

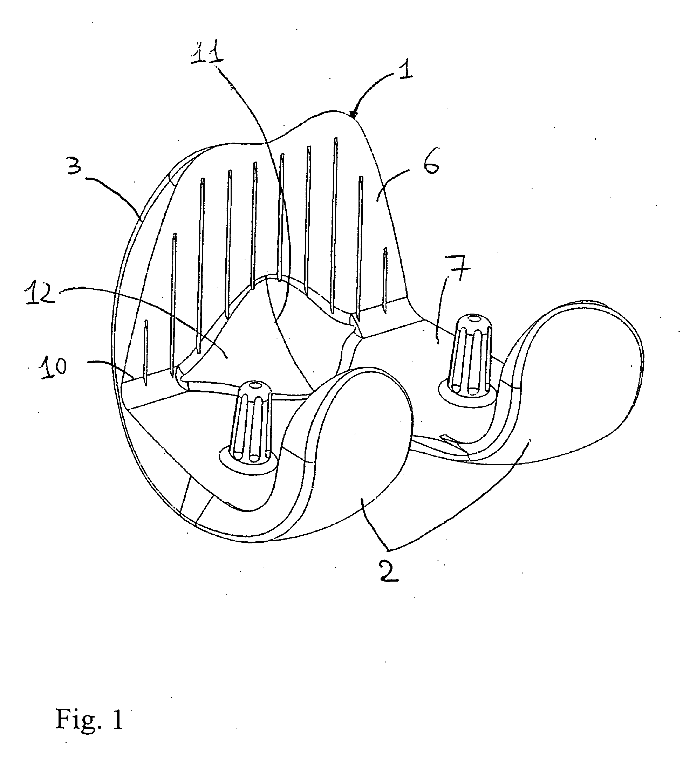

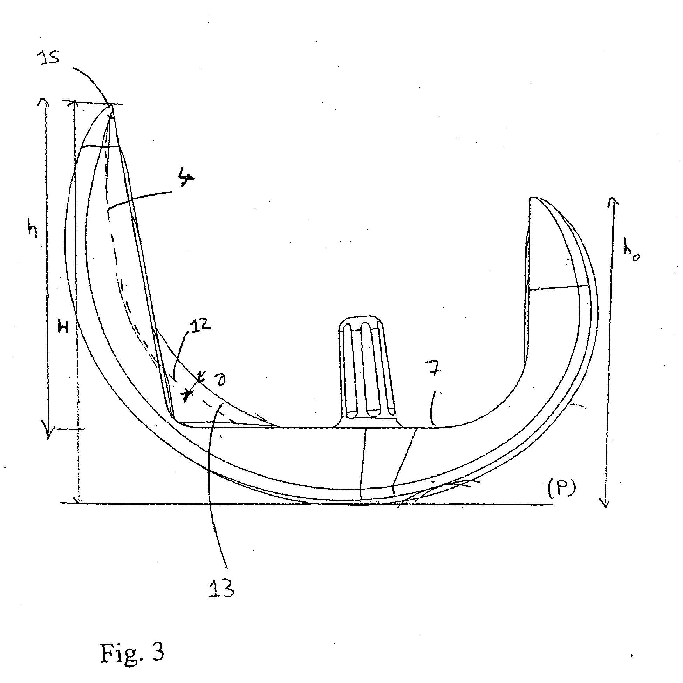

A la Figure 1, il est représenté un composant fémoral d'une prothèse 1 de genou comportant deux condyles 2 et une partie de trochlée 3. Les deux condyles dans le plan antéro-postérieur (plan de la Figure 3) sont définis sensiblement par une courbe circulaire, de même que la partie de trochlée est définie par une autre partie circulaire.

La partie 3 de trochlée dans sa partie extérieure définit un trajet de trochlée 4. Ce trajet de trochlée est formé par un sillon creusé dans la surface extérieure de la partie 3 de trochlée. La partie intérieure du composant fémoral définit une cavité ayant des faces sensiblement planes. Il est ainsi défini une première face 6 plane d'extrémité antérieure, correspondant à la coupe antérieure du fémur, une deuxième face 7 intermédiaire, correspondant à la coupe distale, et une troisième face 8 d'extrémité postérieure, correspondant à la coupe postérieure du fémur. Deux plots d'ancrage à l'os du fémur sur lequel est destiné à être implanté le composant fémoral font saillie de la face 7 intermédiaire. La face 7 intermédiaire est sensiblement perpendiculaire à la face 8 et à la face 6. La cavité intérieure ne comporte que ces trois faces planes. Ainsi, la face 7 intermédiaire est-elle sensiblement contiguë à la face 6 d'extrémité antérieure et à la face 8 d'extrémité postérieure. Une arête 9 définit l'interface entre la face 7 intermédiaire et la face 8 d'extrémité postérieure. Une arête 10 définit l'interface entre la face 7 intermédiaire et la face 6 d'extrémité antérieure.

Entre la face 6 d'extrémité antérieure et la face 7 intermédiaire, il est prévu une surface 12 bombée, dont la convexité est tournée vers l'intérieur de la cavité et la concavité par l'extérieur du composant. En section transversale dans le plan médiolatéral, c'est-à-dire le plan perpendiculaire à l'axe antéro-postérieur et passant par l'axe de symétrie de la prothèse, cette surface 12 bombée a la forme d'une cloche. L'ensemble des sommets de l'ensemble des cloches de chaque section transversale définit une courbe 11 sommet de cette surface 12 bombée. Cette courbe 11 des différents sommets a une concavité tournée vers l'intérieur de la prothèse. Elle est la continuation du trajet de trochlée défini dans la partie 3 de trochlée. En particulier, le trajet 4 de trochlée a un rayon de courbure identique à celui de cette courbe 11 des sommets des cloches.

En section transversale dans le plan correspondant à la face 6 d'extrémité antérieure, la surface 12 bombée s'étend à l'intérieur strictement de la cavité intérieure, de sorte que de part et d'autre de cette surface 12 on peut définir des segments de droite correspondant à l'arête 10 faisant l'interface entre la face 6 d'extrémité antérieure et la face 7 intermédiaire.

Suivant un perfectionnement de l'invention, on peut également prévoir des renforts 13 disposés dans le coin entre la face 8 d'extrémité postérieure et la face 7 intermédiaire. Il peut s'agir notamment d'inserts en forme de paroi ayant un bord supérieur de forme concave, dont la concavité est tournée vers l'intérieur de la prothèse. Ils permettent "d'arrondir" les angles entre la face 7 intermédiaire et les faces 8 d'extrémité postérieures pour éviter une cassure liée à la faible épaisseur de la prothèse dans son ensemble. En effet, maintenant qu'il n'y a que trois faces pour former la cavité intérieure, on peut réaliser la prothèse moins épaisse car on a réussi cependant, grâce à la surface 11 bombée, à obtenir un trajet de trochlée aussi long que dans l'art antérieur. Pour éviter une cassure intempestive de la prothèse, on dispose ainsi des pièces 13 de renfort au niveau du coin entre la ou les faces d'extrémité postérieures et la face 7 intermédiaire. En outre pour que ces inserts ne nécessitent pas de réséquer dans l'os du fémur des fentes de réception, ces inserts sont disposés latéralement aux extrémités medio-latérales des coins.

On définit un plan de référence P parallèle à la face intermédiaire et tangent à la surface extérieure du composant fémoral. On définit la hauteur H du composant fémoral comme étant la distance entre le point 15 le plus éloigné de la partie d'extrémité postérieure et le plan P mesurée le long d'une ligne passant par la face 6 d'extrémité, et la hauteur h de la face 6 d'extrémité la même distance mesurée jusqu'à l'intersection avec la face 7 intermédiaire. Le rapport $\dfrac{h}{H}$ est supérieur ou égal à 0,75.

On définit également la hauteur $h_0$ de la face d'extrémité postérieure parallèlement à cette même face jusqu'à l'intersection avec la face 7 intermédiaire.

Le rapport $\dfrac{h_0}{h}$ est de préférence à 0,6.

**Revendications**

1. Composant fémoral d'une prothèse de genou, comportant au moins un, de préférence deux condyles (2), du côté postérieur ; une partie (3) de trochlée du côté antérieur, dans la surface supérieure de laquelle est défini un trajet (4) de trochlée ; et un ensemble de faces planes intérieures, destinées à venir en contact avec des faces réséquées correspondantes de l'extrémité d'un fémur, définissant une cage ouverte intérieure à la surface intérieure du composant, l'ensemble de faces planes comportant une première face (6) plane d'extrémité antérieure, une deuxième face (7) plane intermédiaire et une troisième face (8) plane d'extrémité postérieure, **caractérisé en ce qu'**il est formé, entre la face plane d'extrémité antérieure et la face plane intermédiaire, une surface (12) bombée ayant sa concavité tournée vers l'extérieur du composant.

2. Composant suivant la revendication 1, **caractérisé en ce que** la surface bombée est symétrique par rapport au plan antéro-postérieur et la courbe définissant la section transversale dans le plan antéro-postérieur de cette surface bombée, au niveau du trajet de la trochlée définie dans la partie de trochlée, est en continuité avec ce même trajet de trochlée, et notamment a le même rayon de courbure et le même centre de courbure.

3. Composant suivant la revendication 1 ou 2, **caractérisé en ce que** la deuxième face plane intermédiaire fait un angle avec la perpendiculaire à la première face plane d'extrémité antérieure d'au moins 4°, de préférence compris entre 6° et 8°.

4. Composant suivant l'une des revendications 1 à 3, **caractérisé en ce que** la deuxième face plane intermédiaire fait un angle avec la perpendiculaire à la troisième face plane d'extrémité postérieure d'au moins 4°, de préférence compris entre 6° et 8°.

5. Composant suivant l'une des revendications 1 à 4, **caractérisé en ce que** la deuxième face plane intermédiaire forme, avec chacune des première et troisième faces planes d'extrémités antérieure et postérieure, des coins, en étant contiguë avec celles-ci.

6. Composant suivant l'une des revendications 1 à 5, **caractérisé en ce que** la surface bombée qui fait saillie à l'intérieur de la cavité a, en coupe dans le plan médiolatéral passant par le plan de la première face plane d'extrémité antérieure, la forme d'une courbe qui est incluse strictement à l'intérieur de la cavité intérieure, de sorte que deux segments de droite de part et d'autre de cette surface bombée forment des arêtes de coin entre la face intermédiaire et la face d'extrémité antérieure.

7. Composant suivant l'une des revendications 1 à 6, **caractérisé en ce qu'**il est disposé dans les coins formés entre la ou les troisième face(s) d'extrémité postérieure(s) et la deuxième face intermédiaire des renforts.

8. Composant suivant l'une des revendications 1 à 7, **caractérisé en ce que** $\dfrac{h`}{H}$ est à 0,75.

9. Composant suivant l'une des revendications 1 à 8, **caractérisé en ce que** $\dfrac{h_o}{h}$ est à 0,6.

10. Prothèse de genou comportant un composant fémoral suivant l'une des revendications 1 à 9, et une partie tibiale, un insert tibial étant disposé entre la partie tibiale et le composant fémoral.

**Claims**

1. Femoral component of a knee prosthesis, comprising at least one, preferably two, condyles (2), on the posterior side; a trochlea part (3) on the anterior side, in the upper surface of which a trochlea trajectory (4) is defined; and a set of planar inner faces intended to come into contact with corresponding resected faces of the end of a femur, defining an open inner cage on the inner surface of the component, all the planar faces comprising a first planar anterior end face (6), a second planar intermediate face (7) and a third planar posterior end face (8), **characterised in that** between the planar anterior end face and the planar intermediate face, a dome-shaped surface (12) is formed with its concavity facing the outside of the component.

2. Component according to claim 1, **characterised in that** the dome-shaped surface is symmetrical in relation to the anteroposterior plane and the curve defining the cross section in the anteroposterior plane of this dome-shaped surface, at the level of the trochlea trajectory defined in the trochlea part, is in continuity with this same trochlea trajectory, and in particular has the same radius of curvature and the same centre of curvature.

3. Component according to claim 1 or 2, **characterised in that** the second planar intermediate face is at an angle of at least 4°, preferably between 6° and 8°, in relation to the perpendicular to the first planar anterior end face.

**4.** Component according to one of the claims 1 to 3, **characterised in that** the second planar intermediate face is at an angle of at least 4°, preferably between 6° and 8°, in relation to the perpendicular to the third planar posterior end face.

**5.** Component according to one of the claims 1 to 4, **characterised in that** the second planar intermediate face forms corners with each of the first and third planar anterior and posterior end faces, being contiguous therewith.

**6.** Component according to one of the claims 1 to 5, **characterised in that** the dome-shaped surface projecting inside the cavity has, in section in the mediolateral plane passing via the plane of the first planar anterior end face, the form of a curve which is included strictly inside the interior cavity in such a way that two straight segments on either side of this dome-shaped surface form corner edge elements between the intermediate face and the anterior end face.

**7.** Component according to one of the claims 1 to 6, **characterised in that** reinforcements are arranged in the corners formed between the third posterior end face(s) and the second intermediate face.

**8.** Component according to one of the claims 1 to 7, **characterised in that** $\dfrac{h}{H}$ is equal to 0.75.

**9.** Component according to one of the claims 1 to 8, **characterised in that** $\dfrac{h_0}{h}$ is equal to 0.6.

**10.** Knee prosthesis comprising a femoral component according to one of the claims 1 to 9, and a tibial part, a tibial insert being arranged between the tibial part and the femoral component.

**Patentansprüche**

**1.** Femurkomponente einer Knieprothese, aufweisend rückseitig wenigstens einen Condylus und vorzugsweise zwei Condyli (2); vorderseitig einen Trochleaabschnitt (3), in dessen Fläche oben eine Trochleakontur (4) ausgebildet ist; und mehrere ebene Innenflächen, die für den Kontakt mit den entsprechenden resizierten Flächen des Oberschenkelknochenendes bestimmt sind und einen offenen Innenhohlraum auf der Innenseite der Komponente bilden, wobei die ebenen Innenflächen eine erste ebene Fläche (6) am Vorderende, eine zweite, ebene Zwischenfläche (7) und eine dritte ebene Fläche (8) am Hinterende aufweisen, **dadurch gekennzeichnet, dass** zwischen der ebenen Fläche am Vorderende und der ebenen Zwischenfläche eine gewölbte Fläche (12) ausgebildet ist, deren konkave Seite von der Komponente nach außen weist.

**2.** Komponente nach Anspruch 1, **dadurch gekennzeichnet, dass** die gewölbte Fläche bezogen auf die anteroposteriore Ebene symmetrisch ist und dass die Kurve, die den Querschnitt in der anteroposterioren Ebene dieser gewölbten Fläche in Höhe der im Trochleaabschnitt ausgebildeten Trochleakontur bildet, sich in der Trochleakontur fortsetzt und insbesondere denselben Krümmungsradius und denselben Krümmungsmittelpunkt aufweist.

**3.** Komponente nach Anspruch 1 oder 2, **dadurch gekennzeichnet, dass** die zweite, ebene Zwischenfläche mit der Senkrechten zur ersten ebenen Fläche am Vorderende einen Winkel von mindestens 4° und vorzugsweise zwischen 6° und 8° einschließt.

**4.** Komponente nach einem der Ansprüche 1 bis 3, **dadurch gekennzeichnet, dass** die zweite, ebene Zwischenfläche mit der Senkrechten zur dritten ebenen Fläche am Hinterende einen Winkel von mindestens 4° und vorzugsweise zwischen 6° und 8° einschließt.

**5.** Komponente nach einem der Ansprüche 1 bis 4, **dadurch gekennzeichnet, dass** die zweite, ebene Zwischenfläche sowohl an die erste als auch an die dritte ebene Fläche am Vorder- bzw. am Hinterende angrenzt und mit diesen eine Ecke bildet.

**6.** Komponente nach einem der Ansprüche 1 bis 5, **dadurch gekennzeichnet, dass** die gewölbte Fläche, die in die Höhlung hineinragt, im Schnittbild in der Mitte-Seite-Ebene, die durch die Ebene der ersten ebenen Fläche am Vorderende geht, die Form einer Kurve hat, die vollständig innerhalb des Innenhohlraums liegt, so dass zwei gerade Segmente beiderseits dieser gewölbten Fläche Eckkanten zwischen der Zwischenfläche und der Fläche am Vorderende bilden.

**7.** Komponente nach einem der Ansprüche 1 bis 6, **dadurch gekennzeichnet, dass** in den Ecken, die zwischen der dritten Fläche bzw. den dritten Flächen am hinteren Ende und der zweiten Zwischenfläche gebildet werden, Verstärkungen vorgesehen sind.

**8.** Komponente nach einem der Ansprüche 1 bis 6,

**dadurch gekennzeichnet, dass** $\dfrac{h}{H}$ 0,75 beträgt.

9. Komponente nach einem der Ansprüche 1 bis 8,

   **dadurch gekennzeichnet, dass** $\dfrac{h_0}{h}$ 0,6 beträgt.

10. Knieprothese mit einer Femurkomponente nach einem der Ansprüche 1 bis 9 und einer Tibiakomponente, wobei zwischen der Tibiakomponente und der Femurkomponente ein Tibiaeinsatz angeordnet ist.

Fig. 1

8

Fig. 2

Fig. 3

Fig. 4

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4081866 A